(19) Europäisches Patentamt
European Patent Office
Office européen des brevets

(11) Veröffentlichungsnummer: **0 278 002 A1**

(12) # EUROPÄISCHE PATENTANMELDUNG

veröffentlicht nach Art. 158 Abs. 3 EPÜ

(21) Anmeldenummer: **87900729.2**

(22) Anmeldetag: **25.08.86**

Daten der zugrundeliegenden internationalen Anmeldung:

(86) Internationale Anmeldenummer:
**PCT/SU 86/00080**

(87) Internationale Veröffentlichungsnummer:
**WO 88/01620 (10.03.88 88/6)**

(51) Int. Cl.⁴: **C 07 D 207/24**

(43) Veröffentlichungstag der Anmeldung: **17.08.88**
**Patentblatt 88/33**

(84) Benannte Vertragsstaaten: **BE CH DE FR GB IT LI SE**

(71) Anmelder: **VSESOJUZNY NAUCHNO-ISSLEDOVATELSKY KHIMIKO-FARMATSEVTICHESKY INST. IMENI SERGO ORDZHONIKIDZE (VNIKHFI), ul. Zubovskaya, 7, Moscow, 119815 (SU)**
Anmelder: **NAUCHNO-ISSLEDOVATELSKY INST. FARMAKOLOGII AKADEMII MEDITSINSKIKH NAUK SSSR, ul. Baltiiskaya, 8, Moscow, 125315 (SU)**

(72) Erfinder: **GRANIK, Vladimir Grigorievich, Konakovsky proezd, 19-64, Moscow, 125565 (SU)**
Erfinder: **STEZHKO, Tatyana Vasilievna, ul. Severnaya, 8-30 Moskovskaya obl., Odintsovo, 143000 (SU)**

(72) Erfinder: **GLUSHKOV, Robert Georgievich, ul. Gorkogo, 43-90, Moscow, 125047 (SU)**
Erfinder: **MASHKOVSKY, Mikhail Davydovich, Leningradsky pr., 75a-55, Moscow, 125057 (SU)**
Erfinder: **ROSCHINA, Lidia Fedorovna, Rostovskaya naberezhnaya, 3-135, Moscow, 119121 (SU)**
Erfinder: **POLEZHAEVA, Antonina Ivanovna, ul. 13-ya Parkovaya, 25-1-6, Moscow, 105215 (SU)**
Erfinder: **PARIMBETOVA, Roza Berkimbaevna, ul. I.Babushkina, 3-217, Moscow, 117292 (SU)**
Erfinder: **BOBKOV, Jury Gennadievich, ul. Latsisa, 33-I-7, Moscow, 123514 (SU)**
Erfinder: **LOSEV, Alexandr Semenovich, ul. Bekhtereva, 7-1-42, Moscow, 115477 (SU)**
Erfinder: **IVANOVA, Irina Alexandrovna, Volokolamskoe shosse, 14-41, Moscow, 125080 (SU)**

(74) Vertreter: **von Füner, Alexander, Dr. et al, Patentanwälte v. Füner, Ebbinghaus, Finck Mariahilfplatz 2 & 3, D-8000 München 90 (DE)**

(54) **1-THIOCARBAMOYLMETHYLPYRROLIDIN-2-THION UND VERFAHREN.**

(57) Die neue Verbindung 1-Thiokarbamoylmethylpyrrolidin-2-thion hat folgende Formel

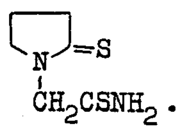

Das Verfahren zur Herstellung der angegebenen Verbindung besteht darin, daß man das Derivat von Pyrrolidon-2 der allgemeinen Formel

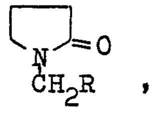
,

worin R für Nitril oder Karbamoyl steht, einer Umsetzung mit Phosphorpentasulfid in einem inerten unpolaren Lösungsmittel bei einer oberhalb der Raumtemperatur liegenden Temperatur unterwirft und anschließend den erhaltenen Niederschlag beim Sieden mit Wasser behandelt und das Endprodukt isoliert.

Die erfindungsgemäße Verbindung besitzt eine antihypoxische und nootrope Wirkung.

EP 0 278 002 A1

ACTORUM AG

# 1-THIOKARBAMOYLMETHYLPYRROLIDIN-2-THION UND VERFAHREN ZUR HERSTELLUNG DESSELBEN

## Gebiet der Technik

Die vorliegende Erfindung bezieht sich auf die organische Chemie und betrifft insbesondere eine neue Verbindung 1-Thiokarbamoylmethylpyrrolidin-2-thion und ein Verfahren zur Herstellung desselben. Die angegebene Verbindung besitzt eine antihypoxische und nootrope Aktivität.

## Der vorherige Stand der Technik

Bekannt sind verschiedene Verbindungen, welche eine antihypoxische und nootrope Aktivität besitzen, es gehört dazu beispielsweise die Verbindung 1-Karbamidomethylpyrrolidon-2 folgender Formel

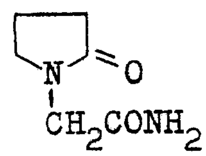,

die als Wirkstoff des Arzneimittels Pyrocetam dient (Mashkovsky M.D., Roschina L.F., Polezhaeva A.I. Nekotorye osobennosti farmakologicheskogo deistviya piratsetama. Einige Besonderheiten der pharmakologischen Wirkung von Pyracetam. Farmakologiya i toksikologiya, 1977, Nr. 6, S. 676-684).

Pyracetam ist ein Hauptvertreter der neuen Klasse von pharmakologisch aktiven Verbindungen Nootropika, deren pharmakologischen Wirkungswerte in einem Schutzeffekt bei unterschiedlichen Arten des Sauerstoffmangels (antihypoxischer Effekt) und einer positiven Beeinflussung der Lern- und Gedächtnisprozesse bestehen.

Pyracetam wirkt als Nootropikum nur bei Applikation in großen Dosen von 500 bis 2000 mg/kg.

Pyracetam wird außerdem dadurch gekennzeichnet, daß es einen ausgeprägten stimulierenden Einfluß auf die Mechanismen der Gedächtniskonsolidierung und Reproduktion von bedingten Reflexen nicht ausübt.

## Offenbarung der Erfindung

Die angemeldete Verbindung ist neu entwickelt und in der Fachliteratur nicht beschrieben.

Zweck der vorliegenden Erfindung ist der, eine neue Verbindung herzustellen, welche eine erhöhte antihypoxische und nootrope Aktivität gleichzeitig aufweist und schwach toxisch ist.

Das Ziel ist dadurch erreicht, daß die angemeldete neue Verbindung 1-Thiokarbamoylmethylpyrrolidin-2-thion gemäß der Erfindung folgende Formel

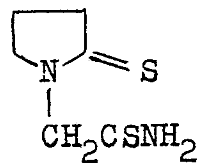

hat.

Die erfindungsgemäße Verbindung stellt eine kristalline Substanz dar, die in N,N-Dimethylformamid löslich ist, aus Wasser, Alkoholen, Äthylazetat, Chloroform, Benzol, Azeton kristallisiert, in Äther, Hexan unlöslich und lagerungsbeständig ist, Schmelzpunkt von 156 bis 158 °C (aus Isopropanol). Die Struktur der Verbindung ist durch Angaben der Elementaranalyse, des IR-Spektrums mit Banden bei Wellenlängen von 1630, 3110 und 3260 $cm^{-1}$ ($NH_2$-Gruppe) und 1120 $cm^{-1}$ (C=S), des Massenspektrums nachgewiesen, welches einen Molekülionpeak 174 m/z enthält und bei dem die Verbindung zwei Schwefelatome hat.

Die beste Ausführungsform

Es waren die Untersuchungen der antihypoxischen und nootropen Aktivität der erfindungsgemäßen Verbindung im Tierversuch durchgeführt.

Die Studie der antihypoxischen Wirkung der erfindungsgemäßen Verbindung verglichen mit Pyracetam erfolgte an verschiedenen experimentellen Modellen der Hypoxiezustände in Mäuse- und Rattenversuchen. Die erfindungsgemäße Verbindung und Pyracetam wurden als wäßrige Emulsion mit Tween 80 intraperitoneal verabreicht.

Es wurden die Untersuchungen am Modell der akuten hypoxischen Hypoxie unter Hyperkapnie durchgeführt.

Zur Untersuchung kamen 540 nicht reinrassige männliche Weißmäuse mit einer Masse von 20 bis 22 g. Man brachte jede Maus in eine hermetische Kammer von 250 ml Fassungsvermögen 60 Minuten nach Gabe der erfindungsgemäßen Verbindung in Dosen von 50, 100, 250, 500 und 750 mg/kg ein. Kontrolltiere erhielten die isotonische Lösung von Natriumchlorid. Die antihypoxische Aktivität wurde nach der Lebensdauer von Mäusen in der hermetischen Kammer (in Minuten) beurteilt.

Ergebnisse, erhalten im Laufe der Untersuchungen der antihypoxischen Wirkung der erfindungsgemäßen Verbindung im Vergleich zu dem bekannten Mittel, Pyracetam, sind in der Tabelle 1 angeführt.

Tabelle 1. Wirkung von zu testenden Verbindungen auf die Lebensdauer von Mäusen (in Minuten) unter Bedingungen der akuten hypoxischen Hypoxie

| Verbindungen | Dosis in mg/kg | | |
|---|---|---|---|
| | 0 | I00 | 250 |
| I | 2 | 3 | 4 |
| Kontrolle | 28,I (22,5-33,7) | - | - |
| erfindungsgemäße Verbindung | - | 56,3 (43,7-68,9) | 60,3 (50,0-70,6) |
| Pyracetam | - | 27,4 (2I,4-33,4) | 33,5 (29,I-37,9) |

Fortsetzung der Tabelle I

| Verbindungen | Dosis in mg/kg | | | |
|---|---|---|---|---|
| | 500 | 750 | I000 | 2000 |
| I | 5 | 6 | 7 | 8 |
| I | - | - | - | - |
| 2 | 95,2 (87,I-I03,3) | II9,2 (II5,7-I22,7) | nicht untersucht | nicht untersucht |
| 3 | 39,6 (35,5-43,7) | 34,4 (29,8-38,0) | 4I,4 (40,2-42,6) | 45,0 (39,I-50,9) |

Wie aus den in der Tabelle 1 angegebenen Prüfdaten zu ersehen ist, vergrößert die erfindungsgemäße Verbindung die Lebensdauer von Mäusen unter Bedingungen der akuten hypoxischen Hypoxie gegenüber Kontrolle schon bei einer Dosis von 100 mg/kg etwa 2fach (um 103,9 %) und bei einer Dosis von 750 mg/kg 4fach (um 324,5 %), was für ihre hohe antihypoxische Aktivität spricht. An ihrer antihypoxischen Wirkung ist die erfindungsgemäße Verbindung dem Pyracetam bedeutend überlegen. So verlängert die erfindungsgemäße Verbindung in einer Dosis von 100 mg/kg die Lebensdauer von Mäusen auf 56,3 min (um 103,9 %) gegenüber Kontrolle und Pyracetam selbst in einer Dosis von 2000 mg/kg, welche die Dosis der erfindungsgemäßen Verbindung 20fache übersteigt, nur auf 45 min (oder um 60 % gegenüber Kontrolle).

Es ist somit am Modell der akuten hypoxischen Hypoxie unter Hyperkapnie festgestellt, daß die erfindungsgemäße Verbindung beginnend von einer Dosis von 100 mg/kg eine Fähigkeit, die Beständigkeit des Tierorganismus gegen akute Hypoxie zu erhöhen, besitzt und die Lebensdauer von Mäusen unter Hypoxiebedingungen verlängert.

Es wurden Untersuchungen am Modell der akuten hypobarischen Hypoxie durchgeführt.

Modelliert wurde die akute hypobarische Hypoxie in einer Durchfluß-Höhenkammer bei einer zwischen 17 und 22 $^{o}C$ liegenden Umgebungstemperatur unter $CO_2$- und Wasseraufnahme. 15 bis 22 g schwere Tiere der einen Generation (männliche Mäuse, Tetrahybride) wurden unter Modellversuchsbedingungen mit einer Durchschnittsgeschwindigkeit von 50 m/s auf eine Höhe von 11000 m gebracht, in welcher ihre Lebensdauer (in Minuten) innerhalb einer Versuchsreihe ermittelt wurde. Mäuse wurden außerdem mit der gleichen Geschwindigkeit auf 10000 m Höhe, in welcher sie 1 Minute lang gehalten wurden, dann auf 11000 m Höhe, wo sie 1 Minute lang gehalten wurden, und so weiter gebracht, bis sie eingingen, wodurch der Höhenmaximum bestimmt wurde. Der Tod von Tieren wurde nach dem Auftreten der Agonalatmung vor dem Hintergrund von Krämpfen fixiert. Für Versuche wurden insgesamt 200 Mäuse verwendet.

Als Vergleichspräparat diente Pyracetam, Die erfindungsgemäße Verbindung und Pyracetam wurden 1 Stunde vor dem Aufstieg eingeführt. Die Versuchsergebnisse wurden statistisch nach der T-Zahl der Student-Modifikation bewertet und auf Kontrolldaten bezogen, welche für 100 % gehalten wurden.

Ähnliche Versuche wurden an nicht reinrassigen männlichen Ratten (180 bis 230 g) in den gleichen Höhenkammern unter Aufstieg auf eine Höhe von 12000 m vorgenommen.

Versuchsergebnisse, gewonnen am Modell der akuten hypobarischen Hypoxie, sind in Tabellen 2 und 3 dargestellt.

Die Versuchsergebnisse zeigen, daß die erfindungsgemäße Verbindung dem Pyracetam an der antihypoxischen Aktivität am angegebenen Modell sowohl in Hinsicht auf die Beeinflussung der Lebensdauer in 11000 m Höhe (3- bis 4mal wirksamer) als

auch in Hinsicht auf die Vergrößerung des Höhenmaximums überlegen ist (die erfindungsgemäße Verbindung vergrößert den Höhenmaximum um 4000 m gegenüber Kontrolle und Pyracetam in einer Dosis von 500 mg/kg).

Tabelle 2. Wirkung der erfindungsgemäßen Verbindung auf die Lebensdauer von Mäusen (in Minuten) in 11000 m Höhe

| Verbindungen | Dosis in mg/kg 0 | 100 | 250 | 500 |
|---|---|---|---|---|
| | Kontrolle | | | |
| Pyracetam | 6,I±2,75<br>I00,0±45,I% | unwirksam | 6,I6±0,72<br>I0I,0±II,8%<br>P > 0,5 | 8,I3±2,43<br>I33,3±39,9%<br>P > 0,5 |
| erfindungsgemäße Verbindung | 6,I±2,75<br>I00,0±45,I% | I2,58±3,64<br>206,2±59,7%<br>0,I < P < 0,2 | I9,97±4,93<br>327,4±80,8%<br>P < 0,05 | 30,0±0<br>49I,8±0,0%<br>P < 0,00I |

Tabelle 3. Wirkung der erfindungsgemäßen Verbindung auf den Höhenmaximum von Mäusen ($H_{max}$ in 1000 m)

| Verbindungen | D o s i s in mg/kg 0 | 250 | 500 |
|---|---|---|---|
| | Kontrolle | | |
| Pyracetam | II,2I± 0,44I | I0,96±0,9I<br>(P > 0,5) | II,55±0,62<br>(P > 0,5) |
| erfindungsgemäße Verbindung | II,2I±0,441 | I4,0±0,707<br>(P < 0,005) | I5,57±0,85<br>(P < 0,00I) |

Es wurden Untersuchungen am Modell der akuten normobarischen Hypoxie durchgeführt.

Die akute normobarische Hypoxie wurde in einer Durchfluß-Höhenkammer bei einer zwischen 17 und 22 °C liegenden Temperatur unter $CO_2$- und Wasseraufnahme modelliert. Das Gasgemisch (97 % $N_2$ und 3 % $O_2$) wurde der Höhenkammer mit einer Geschwindigkeit von 10 l/min zugeführt. Registriert wurde die Lebensdauer von Versuchstieren (Mäuse, Ratten) in den Höhenkammern ab der Zufuhr des Gemisches in die Höhenkammer bis zum Tod, der nach der Agonalatmung vor dem

Hintergrund von Krämpfen bestimmt wurde. Einführungspläne von Präparaten, Tierart und Analyse von Versuchsergebnissen ähneln den oben beschriebenen bei der akuten hypobarischen Hypoxie. Die Versuche wurden an 50 Mäusen und 20 Ratten verwirklicht.

Die Untersuchungsergebnisse sind in der Tabelle 4 dargestellt.

Die gewonnenen Versuchsergebnisse zeugen davon, daß die erfindungsgemäße Verbindung in einer Dosis von 250 mg/kg die Beständigkeit von Mäusen gegen akute normobarische Hypoxie auf das 4,5fache und die von Ratten auf das 1,5fache gegenüber Kontrolle (Unterschiede sind zuverlässig) erhöht und dem Pyracetam an der Wirksamkeit bei der gegebenen Art der Hypoxie in Dosis von 250 mg/kg auf das 5fache und in Dosis von 500 mg/kg auf das 3,2fache bei Mäusen und auf das 1,5fache bei Ratten überlegen ist.

Tabelle 4. Wirkung der erfindungsgemäßen Verbindung auf die Lebensdauer von Versuchstieren (in Minuten) bei der akuten normobarischen Hypoxie

| Tiere | Verbindungen | Dosis in mg/kg 0 | 250 | 500 |
|---|---|---|---|---|
| | | Kontrolle | | |
| Mäuse | Pyracetam | 4,25±0,54<br>I00,0±I2,6% | 3,73±0,254<br>87,7±5,97%<br>(P > 0,5) | 6,08±0,744<br>I43,I±I7,5%<br>(0,05 < P < 0,I |
| | erfindungsgemäße Verbindung | 4,73±0,367<br>I00,0±7,75% | 22,2I±3,23<br>469,5±68,3%<br>(P < 0,00I) | -x |
| Ratten | Pyracetam | I8,6±3,6I<br>I00,0±I9,4% | -<br>umwirksam | I7,3±5,25<br>93,0±2a,2%<br>(P > 0,5) |
| | erfindungsgemäße Verbindung | I8,6±3,6I<br>I00,0±I9,4% | 27,38±7,07<br>I47,2±38,0%<br>(0,I < P < 0,2) | -x |

x) unzweckmäßig, weil der Effekt bei kleineren Dosen erreicht wird.

Es wurden die Untersuchungen am Modell der akuten histotoxischen Hypoxie durchgeführt.

Die akute histotoxische Hypoxie wurde durch subkutane Injektion von Natriumnitroprussid in einer Dosis von 20mg/kg ausgelöst.

Bei diesem Verfahren der Injektion kommt es zum 100%igen Tod von Mäusen innerhalb von 15 bis 25 Minuten nach der Gabe des Präparats. Beurteilt wurde die Lebensdauer von Mäusen nach der Einführung von Natriumnitroprussid vor dem Hintergrund der getesteten Verbindungen (die erfindungsgemäße Verbindung und Pyracetam), die nach dem Atem- und Herzstillstand registriert wurde. Für diese Versuchsreihe benutzte man insgesamt 40 Tiere.

Die Untersuchungsergebnisse sind in der Tabelle 5 angegeben. Sie wurden wie oben beschrieben nach der T-Zahl von Student ermittelt.

Tabelle 5. Wirkung der untersuchten Verbindungen auf die Beständigkeit von Mäusen gegen akute histotoxische Hypoxie (Lebensdauer in Minuten)

| Verbindungen | Dosis in mg/kg | | | |
|---|---|---|---|---|
| | 0<br>Kontrolle | 250 | 500 | 1000 |
| Pyracetam | I9,5±6,55<br>I00,0±33,6% | -<br>unwirksam | I7,98±I,49<br>92,2±7,62%<br>(P>0,5) | 20,97±I,3I<br>I07,6±6,7%<br>(P>0,5) |
| erfindungsgemäße Verbindung | I9,5±6,55<br>I00,0±33,6% | 24,2±2,46<br>I24,I±I2,6%<br>(P<0,5) | 32,2±4,95<br>I65,5±25,4%<br>(0,I<P<0,2) | -X |

x) unzweckmäßig, weil der Effekt bei kleineren Dosen erreicht wird

Die Ergebnisse, gewonnen bei dieser Versuchsreihe, zeugen davon, daß die erfindungsgemaße Verbindung in einer Dosis von 500 mg/kg dem Pyracetam in Dosen von 500 und 1000mg/ auf das 1, 5fache an der Wirksamkeit bei der gegebenen Art der Hypoxie überlegen ist.

Es wurden die Untersuchungen am Modell der akuten wiederholten Anoxie durchgeführt.

Zur Untersuchung kamen 180 bis 200 g schwere Ratten, bei denen Nichrom-Elektroden in die Hirnrinde eingeführt wurden, wobei die Ratten nach der Tracheostomie an ein Beatmungsgerät angeschlossen wurden. Die Anoxie wurde durch wiederholte Atemabschaltungen für 90, 120, 150 und 180 Sekunden mit je Zeitintervall von 10 Minuten hervorgerufen. Aufgenommen wurden Elektrokardiogramme und Elektrokortikogramme. Für die Versuche wurden insgesamt 53 Ratten verwendet. Die antihypoxische Aktivität der erfindungsgemäßen Verbindung verglichen mit Pyracetam beurteilte man nach folgenden Parametern:

I Zeit vor Verschwinden der Hirnaktionsspannung nach dem Abschalten der Sauerstoffzufuhr

II Zeit vor Auftreten des Elektrokortikogramms nach der Wiederbeatmung

III Gesamtzeit, wo die Hirnrinde bei jeder der darauffolgenden Anoxien auf den elektrischen Reiz nicht antwortet.

Die Versuchsergebnisse wurden nach der T-Zahl von Student statistisch beurteilt.

Die Prüfergebnisse sind in Tabellen 6 und 7 angegeben.

Tabelle 6. Wirkung der erfindungsgemäßen Verbindung verglichen mit Pyracetam auf registrierbare Parameter der Hirnaktionsspannung nach der wiederholten Anoxie von verschiedener Dauer

| Verbindungen | Dauer von darauffolgenden Anoxien in Sekunden | | |
|---|---|---|---|
| | 90 | | |
| | Parameter | | |
| | I | II | III |
| I | 2 | 3 | 4 |
| 1. Kontrolle | 45,4±3,8 | 22,4±2,5 | 66,6±6,0 |
| 2. Pyracetam (I000 mg/kg) | 76,7±4,8 xxx | 5,3±0,2 xxx | I7,8±4,9 xxx |
| 3. erfindungsgemäße Verbindung (250 mg/kg) | 84,3±I,8 xxx | 3,7±I,5 xxx | 8,9 ±3,5 xxx |

Fortsetzung der Tabelle 6

| I | 5 | 6 | 7 |
|---|---|---|---|
| I | 48,6±3,3 | 45,4±3,5 | II0,5±7,0 |
| 2 | 85,8±7,3xxx | 2I,8±6,9 xx | 53,3±I2,0 xxx |
| 3 | 89,0±6,2 xxx | I8,3±2,6 xxx | 52,4±5,6 xxx |

x- Unterschiede sind zuverlässig von 0,0I<P ≤ 0,05;
"xx" - 0,00I < P <0,05; "xxx" - P < 0,00I

Fortsetzung der Tabelle 6

| Verbindungen | I50 | | |
|---|---|---|---|
| | Parameter | | |
| | I | II | III |
| I | 8 | 9 | I0 |
| I | 60,I±3,6 | 57,3±I2,6 | I47,9±I5,6 |
| 2 | 95,0±I6,3 x | 67,6±42,I | I3I,2±53,8 |
| 3 | 73,9±7,5 | 64,6±22,2 | I2I,0±25,0 |

Fortsetzung der Tabelle 6

| Verbindungen | I80 Parameter | | |
|---|---|---|---|
| | I | II | III |
| I | II | I2 | I3 |
| I | 76,4±6,9 | - | - |
| 2 | 79,4±23,8 | - | - |
| 3 | 94,4±9,4 | I60,6±83,8 | 2I6,9±72,0 |

"-" Der Parameter wird nicht registriert, weil die Zeit der Wiederaufnahme des Elektrokortikogramms nach der Sauerstoffzufuhr unendlich ist (d.h. die Hirnrinde funktioniert nicht).

Tabelle 7. Wirkung der erfindungsgemäßen Verbindung gegenüber Pyracetam auf die Beständigkeit der Hirnrinde von Ratten gegen asphyxische Hypoxie

| Verbindungen | Zahl von Tieren mit dem erhaltenen Elektrokortikogramm nach der 1. Anoxie (in %) | Zahl von Tieren mit dem wiederaufgenommenen Elektrokortikogramm nach der 4. Anoxie (in %) |
|---|---|---|
| Kontrolle | 8,3 | I5,0 |
| Pyracetam (I000 mg/kg) | I2,5 | I4,3 |
| erfindungsgemäße Verbindung (250 mg/kg) | 44,0 | I00,0 |

Die gewonnenen Ergebnisse zeugen davon, daß die erfindungsgemäße Verbindung in einer Dosis von 250 mg/kg eine ausgeprägte antihypoxische Aktivität bei der asphyxischen Hypoxie besitzt: Die Zeit vor Verschwinden des Elektrokortikogramms durch Wirkung der erfindungsgemäßen Verbindung stieg etwa 2mal insbesondere bei der 1. und 2. Anoxien an, und die Zeit der Wiederherstellung der Funktionsfähigkeit des Gehirns verkürzte sich bedeutend.

Der besonders deutliche antihypoxische Effekt der erfindungsgemäßen Verbindung ist bei der Analyse der Zahl von Tieren mit der nach der 4. Anoxie wiederhergestellten Funktionsaktivität des Gehirns zu beobachten. Während sich die Zahl solcher Tiere in der Kontrolle und bei der Einführung von Pyracetam nur auf 15 % belief, kam es bei der Gabe der erfindungsgemäßen Verbindung in 100 % der Fälle zur Wiederherstellung der Hirnaktionsspannung, was die Wirksamkeit von Pyracetam auf das 7fache überstieg. Die erfindungsgemäße Verbindung zeigte zugleich eine deutliche antihypoxische Wirkung auch in Bezug auf die Zahl von Tieren mit dem nach der 1. 90 Sekunden dauernden Anoxie erhaltenen Elektrokortikogramm, indem sie an ihrer Aktivität dem Pyracetam in einer Dosis von 1000 mg/kg dreimal überlegen war.

Experimente, angestellt mit dem Ziel, die antihypoxische Aktivität der erfindungsgemäßen Verbindung zu untersuchen, haben ergeben, daß die erfindungsgemäße Verbindung die antihypoxische Aktivität bei verschiedenen Formen der hypoxischen Wirkung (an allen verwendeten Modellen) aufweist.

Die erfindungsgemäße Verbindung ist dem Pyracetam an ihrer Aktivität bezüglich des antihypoxischen Effekts in allen Vergleichsfällen überlegen und in kleineren Dosen als Pyracetam wirksam.

Es wurden die Untersuchungen der Wirkung der erfindungsgemäßen Verbindung auf die Lern- und Gedächtnisprozesse durchgeführt.

Die Wirkung der erfindungsgemäßen Verbindung auf die Lerngeschwindigkeit und Reproduktion des bedingten ausgebildeten Reflexes wurde in Versuchen an Ratten unter Verwendung einer Methode von bedingten Vermeidungsreaktionen

auf den elektrischen Reiz untersucht.

In Versuchen an Mäusen wurde die Wirkung der erfindungsgemäßen Verbindung auf die Prozesse der Gedächtniskonsolidierung auf Grund von Untersuchungsbefunden der bedingten Reaktion der passiven Vermeidung geprüft.

Für die Untersuchungen der Wirkung der erfindungsgemäßen Verbindung auf die Lerngeschwindigkeit und Reproduktion des Ausgebildeten bedingten Reflexes verwendete man 40 nicht reinrassige männliche Ratten (4 Gruppen je 10 Ratten), die 150 bis 170 g schwer waren, wobei nach einer Methode von bedingten Vermeidungsreaktionen auf den elektrischen Reiz gearbeitet wurde. Bei allen Ratten wurden bedingte Reflexe auf das Einsetzen des Tons als bedingter Reiz in Kombination mit einem unbedingten Reiz durch elektrischen Strom (40 bis 50 V) ausgebildet, welcher dem elektrifizierten Boden einer bedingtreflektorischen Kammer zugeführt wurde. Als Maß für die Ausbildung des bedingten Reflexes diente der Sprung der Ratte an den Stab in Beantwortung des Tons innerhalb von den ersten 10 Sekunden nach dem Ertönungsbeginn.

Vor dem Hintergrund der Ausbildung der bedingten Reflexe erhielten alle Ratten innerhalb von 3 Wochen (fünfmal je Woche) folgende Präparate: die erste Gruppe die erfindungsgemäße Verbindung in einer Dosis von 250 mg/kg, die zweite und die dritte Gruppe Pyracetam in Dosierung von 500 und 1000 mg/kg und die vierte Gruppe (Kontrollgruppe) die physiologische Kochsalzlösung. Die Applikation erfolgte intraperitoneal 24 Stunden vor Versuchsbeginn.

Es ist festgestellt, daß die erfindungsgemäße Verbindung in einer Dosis von 250 mg/kg eine Beschleunigung der Reproduktion und Festigung des bedingten Reflexes etwa 2mal bewirkt (bei Kontrolltieren wird der bedingte Reflex nach 95 bis 127 Kombinationen von bedingten und unbedingten Reizen und nach 49 bis 65 Reizkombinationen vor dem Hintergrund der Applikation der erfindungsgemäßen Verbindung gefestigt.).

Pyracetam ist in einer Dosis von 500 mg/kg unwirksam, ruft jedoch bei 1000 mg/kg Dosis einen Effekt hervor, der der Wirkung der erfindungsgemaßen Verbindung in einer Dosis

von 250 mg/kg ähnlich ist.

Die erfindungsgemäße Verbindung übt auf die Reproduktion des ausgebildeten bedingten Vermeidungsreflexes auch einen positiven Einfluß aus. Bei einer Dosis von 250 mg/kg tritt die Verkürzung der Latenzzeit des bedingten Reflexes auf mehr als die Hälfte gegenüber Kontrolle auf (bei Kontrollratten beträgt die Latenzzeit 1,43 bis 2,85 Sekunden und 0,55 bis 1,19 Sekunden bei Ratten, welche die erfindungsgemäße Verbindung erhalten haben).

Pyracetam wirkt auf ähnliche Weise (verkürzt die Latenzzeit des Reflexes auf 0,69 bis 1,27 Sekunden) nur in einer Dosis von 1000 mg/kg.

Die erfindungsgemäße Verbindung besitzt somit eine Fähigkeit, den Lernprozeß bei Ratten zu erleichtern und die Reproduktion der bedingten Vermeidungsreaktion zu verbessern, während Pyracetam im Falle der angegebenen Teste weniger wirksam ist (die effektive Dosis der erfindungsgemäßen Verbindung ist 250 mg/kg und die von Pyracetam 1000 mg/kg gleich).

Die Untersuchungen der Wirkung auf die Prozesse der Gedächtniskonsolidierung (Festigung) wurden an 400 nicht reinrassigen männlichen Weißmäusen (18 bis 20 g schwer) unter Verwendung der Methodik des bedingten Reflexes der passiven Vermeidung in einem Versuchsbox aus zwei Kammern vorgenommen, wobei eine der Kammern dunkel ist. Die Sohle der Dunkelkammer verfügt über einen Fußboden mit Elektroden, dem der elektrische Strom (50 bis 60 V) zugeführt werden kann.

Infolge ihrer biologischen Besonderheiten ziehen Mäuse vor, sich in der Dunkelkammer zu befinden. Gegen Ende der 180 Sekunden dauernden Belichtung, falls das Tier in der Dunkelkammer ist, und dies in der Regel doch vorkommt, wird einer Reizvorrichtung der elektrische Strom zugeführt, so daß die Maus gezwungen ist, ihren Zufluchtsort zu verlassen.

Nach dem einmaligen Training wird also bei Mäusen der bedingte Reflex der passiven Vermeidung ausgebildet. Die Reproduktion des Effekts, welcher nach der Latenzzeit (in sek) des Eintritts in die Dunkelkammer (oder nach der Aufenthaltzeit in der Hellkammer) beurteilt wurde, erfolgte nach 24 Stunden.

Die erfindungsgemäße Verbindung und Pyracetam wurden in Dosen von 200 bis 500 mg/kg (Pyracetam selbst in 1000mg/kg Dosis) intraperitoneal sofort nach der Ausbildung des bedingten Reflexes appliziert. Es ist festgestellt, daß die erfindungsgemäße Verbindung in einer Dosis von 200 mg/kg die Latenzzeit des Reflexes auf 129, 6 bis 142,8 Sekunden (bei Kontrolle 95,1 bis 108,9 Sekunden) verlängert. Pyracetam führt zum ähnlichen Effekt der Verlängerung der Latenzzeit des Reflexes (129,8 bis 142,6 Sekunden) nur in 1000mg/kg Dosis.

Sind die gewonnenen Ergebnisse in Prozent (die Latenzzeit des Eintritts in die Dunkelkammer bei Kontrolltieren ist für 100 % zu nehmen) anzugeben, so erhöht sich die Latenzzeit des Reflexes der passiven Vermeidung nach der Injektion der erfindungsgemäßen Verbindung in einer Dosis von 200 mg/kg um 33,0 % und um 33,7 % nach der Injektion von Pyracetam in einer Dosis von 1000 mg/kg. Bei kleinerer Dosierung ist Pyracetam unwirksam. Die Effektivität der positiven Wirkung der erfindungsgemäßen Verbindung steigt mit der Erhöhung der Dosis auf 500 mg/kg nicht.

Die erfindungsgemäße Verbindung beeinflußt somit die Konsolidierungsprozesse (Festigungsprozesse) der erhaltenen Information und wirkt bezüglich dieses Kennwerts in einer Dosis von 200 mg/kg, d.h. in 5mal kleinerer Dosis gegenüber Pyracetam. Die erfindungsgemäße Verbindung verlängert die Latenzzeit des Reflexes der passiven Vermeidung um 33 % im Vergleich zur Kontrolle, während Pyracetam den ähnlichen Effekt in einer Dosis von 1000 mg/kg hervorruft.

Die durchgeführten Versuche haben also ergeben, daß die erfindungsgemäße Verbindung auf die Lerngeschwindigkeit, Reproduktion des ausgebildeten bedingten Reflexes und Prozesse der Gedächtniskonsolidierung positiv wirkt. Die erfindungsgemäße Verbindung ist in Dosen wirksam, die 4- bis 5mal kleiner als die von Pyracetam sind.

Es wurde die Untersuchung der Wechselwirkung der erfindungsgemäßen Verbindung mit Thiosemikarbazid, welches als spezifischer Antagonist der $\gamma$-Aminobuttersäure (GABS) dient, durchgeführt.

Im Zusammenhang damit, daß die $\gamma$-Aminobuttersäure in

der Hirntätigkeit, insbesondere in Lern- und Gedächtnisprozessen eine wichtige Rolle spielt, untersuchte man das mögliche Vorliegen eines GABS-ergischen Bestandteils im Wirkungsmechanismus der erfindungsgemäßen Verbindung. Dazu wurde die Fähigkeit der erfindungsgemäßen Verbindung, Krämpfe zu verhindern, welche durch Thiosemikarbazid als Enzyminhibitor ausgelöst werden, wobei das Enzym die $\gamma$-Aminobuttersäure synthetisiert, geprüft.

Die Versuche führte man an 18 bis 20 g schweren 140 Weißmäusen vor. Thiosemikarbazid wurde in einer Dosis von 20 mg/kg subkutan appliziert.

Die erfindungsgemäße Verbindung wurde in die Bauchhöhle bei Dosishöhen von 50, 250 und 500 mg/kg 20 Minuten nach Applikation von Thiosemikarbazid injiziert.

Je Dosis wurden 20 Mäuse genommen. Die Wirkung gegen Krampfzustände beurteilte man nach der Änderung der Latenzzeit vor Krampfanfall und Verlängerung des Zeitraums vor dem Tod von Tieren. Die Versuche wurden im Vergleich zu Pyracetam durchgeführt. Die gewonnenen Angaben sind in der Tabelle 8 angegeben.

Tabelle 8. Wirkung der erfindungsgemäßen Verbindung und Pyracetam auf Krämpfe, ausgelöst durch Thiosemikarbazid

| Verbindungen | Dosis in mg/kg | Latenzzeit vor Krampfanfall (in Minuten) | Zeitraum vor dem Tod von Tieren ( in Minuten) |
|---|---|---|---|
| Kontrolle (0,9%ige NaCl-Lösung) | - | 63,5(57,I-69,9) | 69,5(65,8-73,2) |
| erfindungsgemäße Verbindung | 50 | 59(5I,5-66,5) | 76(64-88) |
| | 250 | 82,6(76,I-89,I) | I00,8(93,5-I08,I) |
| | 500 | 80(75,6-86,4) | I05(95-II5) |
| Pyracetam | I000 | 66,6(6I,0-72,2) | 79,9(64-95,8) |
| | 2000 | 70,6(65,6-75,6) | 82(73,6-90,4) |

Wie aus der Tabelle zu ersehen ist, verlängert die erfindungsgemäße Verbindung, injiziert in Dosen von 250 und 500 mg/kg in die Bauchhöhle, gegenüber Kontrolle die Latenzzeit vor Krampfanfall um 25 bis 30 % und vergrößert den Zeitraum vor dem Tod um 45 bis 50 %.

Pyracetam, appliziert in einer Dosis, welche die vierfache Dosis der erfindungsgemäßen Verbindung beträgt, verlängert ebenfalls gegenüber Kontrolle die Latenzzeit vor Krampfanfall nur um 10 % und vor dem Tod um 17 %.

Die erhaltenen Angaben zeugen davon, daß die GABSergischen Hirnstrukturen am Wirkungsmechanismus der erfindungsgemäßen Verbindung teilnehmen können.

Bei Pyracetam kommt der ähnliche Effekt sehr schwach und in höheren Dosen zum Ausdruck.

Die Untersuchung der Toxizität und Allgemeinwirkung der erfindungsgemäßen Verbindung erfolgte an 18 bis 20 schweren Mäusen unter intraperitonealer Applikation der erfindungsgemäßen Verbindung in einer Dosis von 100 bis 1500 mg/kg.

Es ist festgestellt, daß die Unterdrückung des Allgemeinzustands von Tieren ab Dosis von 250 mg/kg beginnt. Im Falle der erfindungsgemäßen Verbindung beträgt $LD_{50}$ 1200 mg/kg bei intraperitonealer Applikation und 1900 mg/kg bei intravenöser Verabreichung.

Es sei zu vermerken, daß zu schwach toxischen Verbindungen Substanzen gehören, deren $LD_{50}$-Wert 1000 mg/kg übersteigt, wodurch die erfindungsgemäße Verbindung als schwach toxisch angesehen werden kann.

Gegenstand der Erfindung ist ebenfalls ein Verfahren zur Herstellung der erfindungsgemäßen Verbindung.

Das Verfahren zur Herstellung einer neuen Verbindung 1-Thiokarbamoylmethylpyrrolidin-2-thion besteht erfindungsgemäß darin, daß man die Derivate von Pyrrolidon-2 der allgemeinen Formel

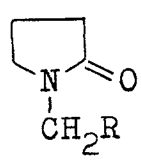

worin R für Nitril oder Karbamoyl steht, einer Umsetzung mit Phosphorpentasulfid in einem inerten unpolaren Lösungsmittel bei einer oberhalb der Raumtemperatur liegenden Temperatur unterwirft und anschließend den erhaltenen Niederschlag mit Wasser beim Sieden behandelt und das Endprodukt isoliert.

Als inertes unpolares Lösungsmittel dient bevorzugt Xylol oder Benzol.

Zur Steigerung der Ausbeute an Endprodukt ist der Prozeß bei einer zwischen 80 und 145 °C liegenden Temperatur zweckmäßigerweise durchzuführen.

Der Prozeß wird nach folgendem Schema durchgeführt:

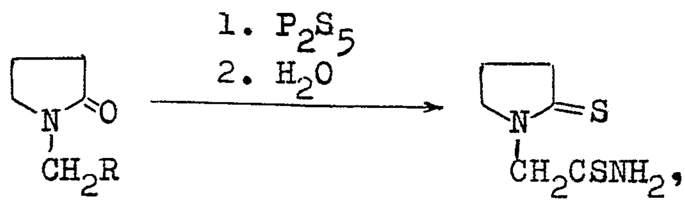


worin R für CN- , $CONH_2$- steht.

Das Derivat von Pyrrolidon-2 der genannten allgemeinen Formel behandelt man mit Phosphorpentasulfid in einem inerten unpolaren Lösungsmittel bei einer oberhalb der Raumtemperatur vorzugsweise zwischen 80 und 145 °C liegenden Temperatur.

Als inertes unpolares Lösungsmittel läßt sich ein beliebiges dazu geeignetes Lösungsmittel zum Beispiel Mesitylen, Toluol, vorzugsweise Xylol, Benzol verwenden. Der durch Reaktion gewonnene Niederschlag wird beim Sieden mit Wasser behandelt. Das erhaltene Reaktionsgut filtriert man, kühlt die Mutterlauge ab, trennt das ausgefallene Endprodukt ab.

Die Ausbeute an Endprodukt beträgt höchstens 65 Gew.%, Schmelzpunkt 156 bis 158 °C (aus Isopropanol).

Zum besseren Verstehen der vorliegenden Erfindung werden folgende Beispiele zur Ausführung des Verfahrens zur Herstellung von 1-Thiokarbamoylmethylpyrrolidin-2-thion angeführt.

Beispiel 1

Eine Suspension, bestehend aus 21,6 g (0,175 Mol) 1-Zyanomethyl-2-oxopyrrolidin, 37,9 g (0,175 Mol) Phosphorpentasulfid und 850 ml trockenem Xylol, wird bei einer Temperatur von 130 °C innerhalb von 4 Stunden erhitzt. Man kühlt das Gemisch auf Raumtemperatur ab, filtriert den Niederschlag ab und kocht in 2500 ml Wasser innerhalb von 30 Minuten.

Das Reaktionsgut wird abfiltriert, die Mutterlauge bei einer zwischen 8 und 10 °C liegenden Temperatur abgekühlt

und das ausgefällene Produkt abfiltriert. Man erhält 14,8 g 1-Thiokarbamoylmethylpyrrolidin-2-thion, Schmelzpunkt 154 bis 156 °C.

Man dampft die Mutterlauge ein und erhält zusätzlich 4,8 g Endprodukt, Schmelzpunkt 153 bis 155 °C.

Die Gesamtausbeute an 1-Thiokarbamoylmethylpyrrolidin-2-thion beträgt 19,6 g (64,5 Gew.%).

Zwecks Analyse wird das Endprodukt aus Isopropanol kristallisiert. Der Schmelzpunit liegt zwischen 156 und 158 °C.

Gefunden in %: C 41,46; H 5,74; N 16,33; S 36,81, $C_6H_{10}N_2S_2$.

Berechnet in %: C 41,35; H 5,78; N 16,07; S 36,80. IR-Spektrum, $\nu$, $cm^{-1}$: 1630, 3110, 3260 $cm^{-1}$ ($NH_2$); 1120 $cm^{-1}$ (C = S).

Beispiel 2

Unter Bedingungen, welche denen in Beispiel 1 ähneln, nur daß als Lösungsmittel Toluol dient und der Prozeß bei 110 °C erfolgt, wird das Endprodukt in einer Ausbeute von 49 Gew.% mit Schmelzpunkt von 154 bis 156 °C erhalten.

Beispiel 3

Unter Bedingungen, welche denen in Beispiel 1 ähneln, nur daß als Lösungsmittel Mesitylen dient und der Prozeß bei einer zwischen 140 und 145 °C liegenden Temperatur erfolgt, wird das Endprodukt in einer Ausbeute von 41 Gew.% mit Schmelzpunkt von 154 bis 156 °C erhalten.

Beispiel 4

Unter Bedingungen, welche denen in Beispiel 1 ähneln, nur daß als Lösungsmittel Benzol dient und der Prozeß bei 80 °C erfolgt, wird das Endprodukt in einer Ausbeute von 62 Gew.% mit Schmelzpunkt von 154 bis 156 °C erhalten.

Beispiel 5

Eine Suspension, bestehend aus 2,8 g (0,02 Mol) 1-Karbamoylmethylpyrrolidon-2 und 8,8 g (0,04 Mol) Phosphorpentasulfid und 50 ml trockenem Xylol, wird innerhalb von 4 Stunden bei 130 °C erhitzt.

Man kühlt das Gemisch ab, filtriert den Niederschlag ab und kocht innerhalb von 30 Minuten in 150 ml Wasser. Das

Reaktionsgut wird abfiltriert, die Mutterlauge bei einer zwischen 8 und 10 °C liegenden Temperatur abgekühlt, das ausgefallene Produkt abfiltriert.

Man erhält 1,1 g (32 Gew.%) 1-Thiokarbamoylmethylpyrrolidin-2-thion mit Schmelzpunkt von 154 bis 156 °C.

Industrielle Anwendbarkeit

Die erfindungsgemäße Verbindung besitzt eine antihypoxische und nootrope Aktivität und kann in der Medizin zur Prophylaxe und Therapie von Zuständen Verwendung finden, welche von der Hypoxie begleitet oder hypoxiebedingt werden: Ischämie von Organen und Geweben (Hirn- und Herzischämie), Wiederherstellung nach ischämischen Beschädigungen und Zuständen, Chirurgie, Therapie von Infarkten und Insulten, antiischämische Konservierung von Organen und Geweben zwecks ihrer Transplantation, Gerontologie, neurologisches Defizit, Prophylaxe und Therapie von Hypoxien bei gesunden Menschen bei Bergbesteigung, bei Vergiftungen mit Prohypoxanten (Methämoglobinbildner und Blocker der Atemkette).

PATENTANSPRÜCHE:

1. 1-Thiokarbamoylmethylpyrrolidin-2-thion folgender Formel

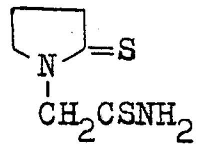

2. Verfahren zur Herstellung von 1-Thiokarbamoylmethyl-pyrrolidin-2-thion, dadurch gekennzeichnet, daß man ein Derivat von Pyrrolidon-2 der allgemeinen Formel

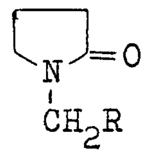 ,

worin R für Nitril oder Karbamoyl steht, einer Umsetzung mit Phosphorpentasulfid in einem inerten unpolaren Lösungsmittel bei einer oberhalb der Raumtemperatur liegenden Temperatur unterwirft und anschließend den erhaltenen Niederschlag beim Sieden mit Wasser behandelt und das Endprodukt isoliert.

3. Verfahren nach Anspruch 2, dadurch gekennzeichnet, daß als inertes unpolares Lösungsmittel Xylol oder Benzol dient.

4. Verfahren nach Anspruch 2 bis 3, dadurch gekennzeichnet, daß der Prozeß bei einer zwischen 80 und 145 °C liegenden Temperatur erfolgt.

# INTERNATIONAL SEARCH REPORT

00278002

International Application No PCT/SU 86/00080

**I. CLASSIFICATION OF SUBJECT MATTER** (if several classification symbols apply, indicate all) [8]

According to International Patent Classification (IPC) or to both National Classification and IPC

$IPC^4$ C07D 207/24

**II. FIELDS SEARCHED**

Minimum Documentation Searched [7]

| Classification System | Classification Symbols |
|---|---|
| $IPC^4$ | C07D 207/24 |

Documentation Searched other than Minimum Documentation to the Extent that such Documents are Included in the Fields Searched [8]

**III. DOCUMENTS CONSIDERED TO BE RELEVANT** [9]

| Category [*] | Citation of Document, [11] with indication, where appropriate, of the relevant passages [12] | Relevant to Claim No. [13] |
|---|---|---|
| A | Farmakologia i toxikologia, Nr. 6, 1977, (Meditsina, Moscow), M.D. Mashkovsky et al "Nekotorye osobennosti farmakologichesko-go deistvia piratsetama" see pages 676-684 | 1 |
| A | SU, A3, 243516 (Union chimique-Chemische Bedrigven) 30 September·1969 (30.09.69) see example 1 | 1 |
| A | Methoden der Organischen Chemie (Houben-Weyl), X1/2, 1958 (Georg Thieme Verlag, Stuttgart) see page 575 | 2-4 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 05 March 1987 (05.03.87) | 20 May 1987 (20.05.87) |
| **International Searching Authority** | **Signature of Authorized Officer** |
| ISA/SU | |